# EUROPEAN PATENT SPECIFICATION

(11) **EP 3 636 295 B1**
(45) Date of publication and mention of the grant of the patent: **26.07.2023**
(21) Application number: 18810635.5
(22) Date of filing: 30.05.2018
(51) Int. Cl.: A61M 1/16, A61M 1/34, A61M 1/36

(54) **BLOOD PURIFICATION APPARATUS**
BLUTREINIGUNGSVORRICHTUNG
DISPOSITIF DE PURIFICATION DU SANG

(30) Priority: 01.06.2017 JP 2017109426
(43) Date of publication of application: 15.04.2020
(73) Proprietor: Nikkiso Co., Ltd., Shibuya-ku, Tokyo 150-6022 (JP)
(72) Inventor: KATAYAMA, Yuki, Makinohara-shi Shizuoka 421-0496 (JP); TAKEUCHI, Satoshi, Makinohara-shi Shizuoka 421-0496 (JP); MAKI, Hideto, Makinohara-shi Shizuoka 421-0496 (JP)
(74) Representative: von Hirschhausen, Helge
(86) International application number: PCT/JP2018/020762
(87) International publication number: WO 2018/221585

(56) References cited:
- WO-A2-2012/161744
- JP-A- 2004 049 492
- JP-A- 2004 049 492
- US-A1- 2006 226 079
- US-A1- 2012 247 567
- US-B2- 8 496 807

## Description

### Technical Field

The present invention relates to a blood purification apparatus for giving blood purification treatment by purifying blood of a patient.

### Background Art

A typical dialysis apparatus as a blood purification apparatus intended for dialysis treatment or the like includes an arterial blood circuit provided at the distal end thereof with an arterial puncture needle through which blood is collected from a patient, and a venous blood circuit provided at the distal end thereof with a venous puncture needle through which the blood is returned to the patient. The arterial blood circuit and the venous blood circuit are connected to each other through a dialyzer serving as a blood purifier. While the blood of the patient is caused to extracorporeally circulate through the arterial blood circuit and the venous blood circuit, the dialyzer can perform dialysis treatment (blood purification treatment).

If, for example, the condition of the patient is changed significantly or the patient needs to take a bathroom break during the blood purification treatment, the treatment needs to be suspended so that the patient is released from the blood circuit (the puncture needles are dislodged from the patient). To suspend the blood purification treatment in such a known blood purification apparatus, as disclosed by PTL 1 for example, the patient is only allowed to be released from the blood circuit after a process of blood return in which the blood in the blood circuit is returned to the patient.

### Citation List

### Patent Literature

PTL 1: Japanese Unexamined Patent Application Publication No. 2016-27901

### Summary of Invention

### Technical Problem

To restart the blood purification treatment in the known blood purification apparatus by causing the blood of the patient to extracorporeally circulate through the blood circuit after the suspension of the blood purification treatment with the release of the patient from the blood circuit during the blood purification treatment, values of some settings, such as the amount of ultrafiltration and the rate of ultrafiltration, determined at the start of the treatment need to be changed, depending on the amount of water, such as substitution fluid, introduced for blood return. Typically, such change of setting values is performed manually by medical staff.

In particular, after the suspension for the patient to take a bathroom break, the body weight of the patient may decrease with urination or defecation. Moreover, if the patient has taken any food or drink during the suspension, the patient's body weight may increase by the weight of the food or drink. Therefore, parameters related to ultrafiltration need to be changed with consideration for the increase or decrease in the patient's body weight that may occur during the suspension. On the other hand, when the patient is released from the blood circuit, emergency fluid infusion may be performed. Therefore, the parameters related to ultrafiltration may need to be changed with consideration for the amount of infusion fluid as well. Accordingly, the known blood purification apparatus accompanies complicated work in changing the setting values of the parameters related to ultrafiltration, and such circumstances may trigger wrong setting of the parameters related to ultrafiltration.

The present invention has been conceived in view of the above circumstances and provides a blood purification apparatus in which parameters related to ultrafiltration can be changed assuredly and smoothly at the restart of blood purification treatment suspended with the release of the patient from a blood circuit during the blood purification treatment.

### Solution to Problem

According to the invention of Claim 1, there is provided a blood purification apparatus including a blood circuit through which blood of a patient is allowed to extracorporeally circulate; a blood purifier for giving blood purification treatment by purifying the blood of the patient that extracorporeally circulates through the blood circuit; an ultrafiltration device capable of performing ultrafiltration for removing water from liquid in the blood circuit; an infusion device capable of performing fluid infusion or blood return by introducing liquid into the blood circuit; a setting device capable of setting a parameter related to ultrafiltration performed by the ultrafiltration device; and a control device that controls the ultrafiltration device in accordance with the parameter that is set by the setting device. The blood purification treatment that is suspended with a release of the patient from the blood circuit during the blood purification treatment is restartable. The blood purification apparatus further includes a calculating device capable of calculating a variable amount related to the patient's body weight that changes with the suspension and restart of the blood purification treatment; and a changing device capable of changing, in accordance with the variable amount calculated by the calculating device, the parameter related to ultrafiltration that is set by the setting device.

According to the invention of Claim 2, in the blood purification apparatus according to Claim 1, the variable amount related to the patient's body weight that is calculated by the calculating device is one of variable amounts including an amount of liquid delivered to the blood circuit during fluid infusion or blood return performed by the infusion device at the suspension of the blood purification treatment, or an amount of water removed from the blood circuit during ultrafiltration performed by the ultrafiltration device in a process of blood removal at the restart of the blood purification treatment.

According to the invention of Claim 3, in the blood purification apparatus according to Claim 2, the variable amount related to the patient's body weight that is calculated by the calculating device is one of variable amounts including the amount of liquid delivered by the infusion device, an increment in the body weight that is attributed to food and drink taken by the patient, the amount of water removed by the ultrafiltration device, and a decrement in the body weight that is attributed to urination or defecation by the patient.

According to the invention of Claim 4, in the blood purification apparatus according to Claim 3, the patient's body weight at the suspension of the blood purification treatment and the patient's body weight at the restart of the blood purification treatment are inputtable, and a change in the patient's body weight is calculable from the inputted body weights.

According to the invention of Claim 5, in the blood purification apparatus according to any of Claims 1 to 4, any variable amount related to the patient's body weight that is calculated by the calculating device is inputtable through a piece of equipment linked to a body of the apparatus or through manual operation by an operator.

According to the invention of Claim 6, in the blood purification apparatus according to any of Claims 1 to 5, a total volume of a liquid flow route in the blood purifier or the blood circuit is inputtable, and if the blood purifier or the blood circuit is exchanged at the restart of the blood purification treatment, the variable amount related to the patient's body weight is calculable in accordance with the inputted total volume.

### Advantageous Effects of Invention

According to the invention of Claim 1, the blood purification apparatus includes the calculating device capable of calculating the variable amount related to the patient's body weight that changes with the suspension and restart of the blood purification treatment; and the changing device capable of changing, in accordance with the variable amount calculated by the calculating device, the parameter related to ultrafiltration that is set by the setting device. Therefore, the parameter related to ultrafiltration can be changed assuredly and smoothly at the restart of the blood purification treatment suspended with the release of the patient from the blood circuit during the blood purification treatment.

According to the invention of Claim 2, the variable amount related to the patient's body weight that is calculated by the calculating device is one of variable amounts including the amount of liquid delivered to the blood circuit during fluid infusion or blood return performed by the infusion device at the suspension of the blood purification treatment, or the amount of water removed from the blood circuit during ultrafiltration performed by the ultrafiltration device in the process of blood removal at the restart of the blood purification treatment. Therefore, the parameter related to ultrafiltration can be changed more assuredly and smoothly.

According to the invention of Claim 3, the variable amount related to the patient's body weight that is calculated by the calculating device is one of variable amounts including the amount of liquid delivered by the infusion device, the increment in the body weight that is attributed to food and drink taken by the patient, the amount of water removed by the ultrafiltration device, and the decrement in the body weight that is attributed to the urination or defecation by the patient. Therefore, the parameter related to ultrafiltration can be changed much more assuredly and smoothly.

According to the invention of Claim 4, the patient's body weight at the suspension of the blood purification treatment and the patient's body weight at the restart of the blood purification treatment are inputtable, and the change in the patient's body weight is calculable from the inputted body weights. Therefore, the parameter related to ultrafiltration can be changed in accordance with the change in the body weight that cannot directly be grasped by the blood purification apparatus.

According to the invention of Claim 5, any variable amount related to the patient's body weight that is calculated by the calculating device is inputtable through the piece of equipment linked to the body of the apparatus or through manual operation by the operator. Therefore, the parameter related to ultrafiltration can be changed in accordance with any variable amount related to the body weight that cannot directly be grasped by the blood purification apparatus.

According to the invention of Claim 6, the total volume of the liquid flow route in the blood purifier or the blood circuit is inputtable. Furthermore, if the blood purifier or the blood circuit is exchanged at the restart of the blood purification treatment, the variable amount related to the patient's body weight is calculable in accordance with the inputted total volume. Therefore, even if the blood purifier or the blood circuit is exchanged at the restart of the blood purification treatment, the parameter related to ultrafiltration can be changed assuredly.

### Brief Description of Drawings

[Fig. 1] Fig. 1 is a diagram illustrating a blood purification apparatus according to an embodiment of the present invention.
[Fig. 2] Fig. 2 is a perspective view of a body of the blood purification apparatus.
[Fig. 3] Fig. 3 is a diagram illustrating information displayed on a display of the blood purification apparatus (before body weight is inputted).
[Fig. 4] Fig. 4 is a diagram illustrating an input device included in the blood purification apparatus.
[Fig. 5] Fig. 5 is a diagram illustrating information displayed on the display of the blood purification apparatus (after body weight is inputted).
[Fig. 6] Fig. 6 is a flow chart illustrating a method of changing parameters related to ultrafiltration in the blood purification apparatus.
[Fig. 7] Fig. 7 is a flow chart illustrating a method of changing parameters related to ultrafiltration in a blood purification apparatus according to another embodiment of the present invention.
[Fig. 8] Fig. 8 is a diagram illustrating information displayed on a display of a blood purification apparatus according to another embodiment of the present invention.
[Fig. 9] Fig. 9 is a diagram illustrating a blood purification apparatus according to another embodiment of the present invention.
[Fig. 10] Fig. 10 is a diagram illustrating a blood purification apparatus according to yet another embodiment of the present invention.

### Description of Embodiments

Embodiments of the present invention will now be described specifically with reference to the drawings.

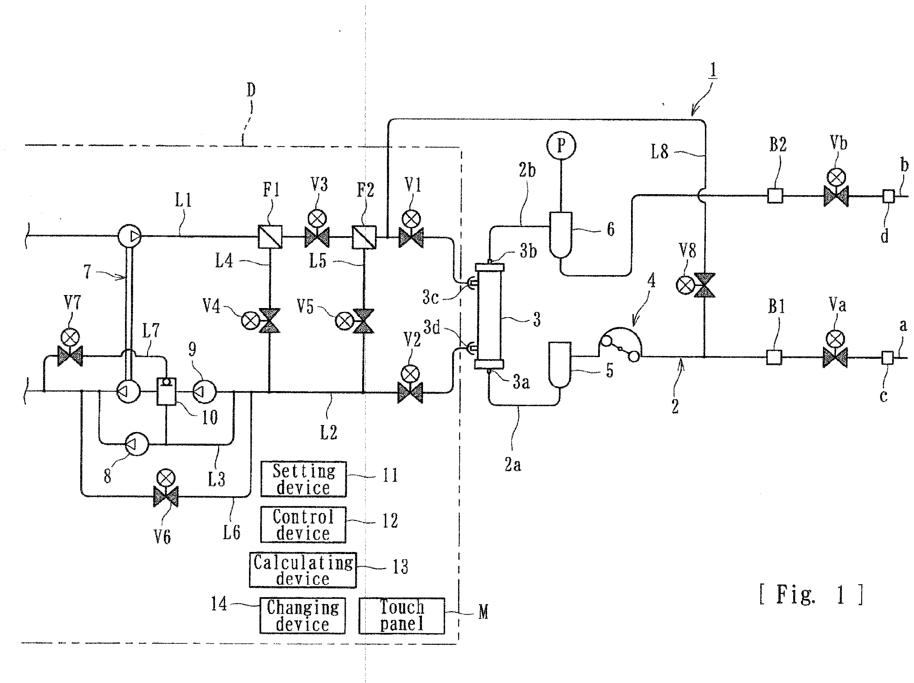

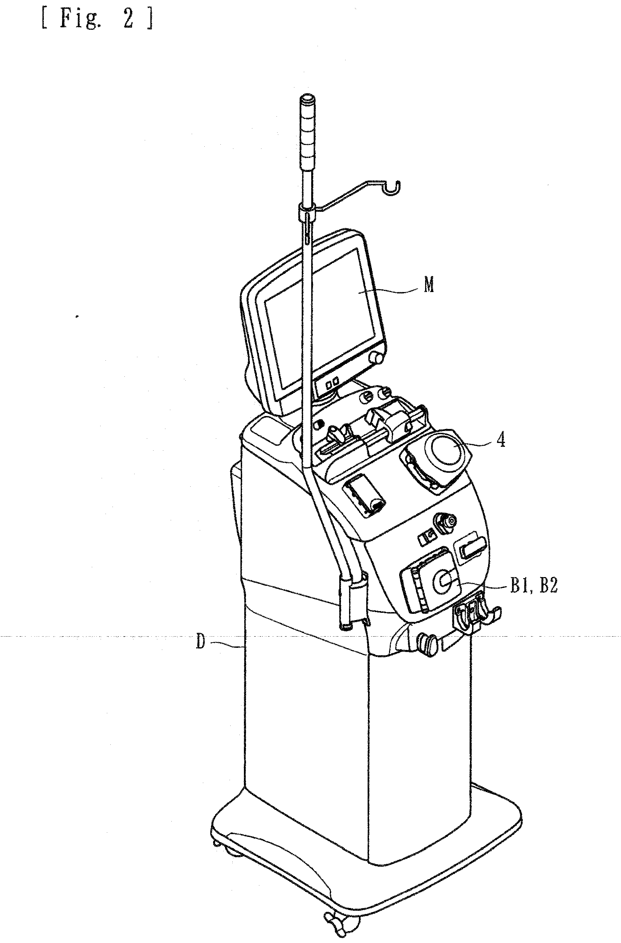

A blood purification apparatus according to an embodiment is applied to a blood purification apparatus 1 in which blood of a patient that is in extracorporeal circulation is purified for dialysis treatment (blood purification treatment). As illustrated in Figs. 1 and 2, the blood purification apparatus includes a blood circuit 2 including an arterial blood circuit 2a and a venous blood circuit 2b, a dialyzer 3 as a blood purifier, a body D provided with tubes such as a dialysate introduction line L1 and a dialysate drain line L2, a touch panel M as a display, a setting device 11, a control device 12, a calculating device 13, and a changing device 14.

The arterial blood circuit 2a is provided at the distal end thereof with a connector c to which an arterial puncture needle a is connectable, and at halfway positions thereof with a peristaltic blood pump 4 and an arterial air-trap chamber 5, respectively. The venous blood circuit 2b is provided at the distal end thereof with a connector d to which a venous puncture needle b is connectable, and at a halfway position thereof with a venous air-trap chamber 6.

The venous air-trap chamber 6 is provided with a venous pressure sensor P capable of detecting venous pressure in accordance with the hydraulic pressure in the venous blood circuit 2b, whereby the hydraulic pressure (venous pressure) of the blood that is in extracorporeal circulation can be detected through the venous pressure sensor P. Hence, the venous pressure of the blood extracorporeally circulating through the blood circuit 2 can be monitored, so that any changes in the condition of the patient during the treatment can be grasped.

The dialyzer 3 (the blood purifier) has, in a housing thereof, a blood inlet 3a (a blood introduction port), a blood outlet 3b (a blood delivery port), a dialysate inlet 3c (a dialysate-flow-route inlet: a dialysate introduction port), and a dialysate outlet 3d (a dialysate-flow-route outlet: a dialysate delivery port). The arterial blood circuit 2a is connected to the blood inlet 3a, and the venous blood circuit 2b is connected to the blood outlet 3b. The dialysate inlet 3c and the dialysate outlet 3d are connected to the dialysate introduction line L1 and the dialysate drain line L2, respectively.

The dialyzer 3 houses a plurality of hollow fibers (not illustrated). The hollow fibers serve as blood purification membranes for purifying blood. The dialyzer 3 has thereinside blood flow routes (flow routes extending between the blood inlet 3a and the blood outlet 3b) in which blood of the patient flows along the blood purification membranes, and dialysate flow routes (flow routes extending between the dialysate inlet 3c and the dialysate outlet 3d) in which dialysate flows. The hollow fibers serving as blood purification membranes each have a number of very small holes (pores) extending therethrough from the outer peripheral surface to the inner peripheral surface, thereby forming the hollow fiber membranes. Impurities and the like contained in the blood are allowed to penetrate through the membranes into the dialysate.

When the blood pump 4 is activated with the arterial puncture needle a provided at the distal end of the arterial blood circuit 2a and the venous puncture needle b provided at the distal end of the venous blood circuit 2b being stuck in the patient, blood of the patient flows through the arterial blood circuit 2a while bubbles generated therein are removed (bubble removal) in the arterial air-trap chamber 5, and reaches the dialyzer 3, where the blood undergoes blood purification treatment. Then, the blood flows through the venous blood circuit 2b while bubbles generated therein are removed (bubble removal) in the venous air-trap chamber 6, and returns into the body of the patient. Thus, the blood of the patient can be purified by the dialyzer 3 while being caused to extracorporeally circulate through the blood circuit 2 from the distal end of the arterial blood circuit 2a to the distal end of the venous blood circuit 2b.

A distal part (a part near the connector c) of the arterial blood circuit 2a and a distal part (a part near the connector d) of the venous blood circuit 2b according to the present embodiment are provided with respective bubble detection devices (B1 and B2) that are each capable of detecting any gas (bubbles) in the blood flowing through a corresponding one of the arterial blood circuit 2a and the venous blood circuit 2b during the blood purification treatment. Furthermore, the distal part (the part near the connector c) of the arterial blood circuit 2a and the distal part (the part near the connector d) of the venous blood circuit 2b are provided with respective clamping devices Va and Vb that are each capable of closing or opening a corresponding one of flow routes by being opened or closed.

The dialysate introduction line L1 and the dialysate drain line L2 are provided with a duplex pump 7 that delivers dialysate prepared to have a predetermined concentration to the dialyzer 3 and discharges waste products and the like (waste liquid) together with the dialysate from the dialyzer 3. Specifically, the duplex pump 7 is provided over the dialysate introduction line L1 and the dialysate drain line L2. When the duplex pump 7 is activated, the dialysate is allowed to be introduced into the dialyzer 3 through the dialysate introduction line L1 while being drained (as waste liquid) from the dialyzer 3 through the dialysate drain line L2.

The dialysate introduction line L1 is provided with electromagnetic valves V1 and V3 and filters F1 and F2. Hence, the dialysate to be introduced into the dialyzer 3 can be filtered through the filters F1 and F2, and the flow route can be closed or opened with any timing at the electromagnetic valves V1 and V3. The dialysate introduction line L1 is connected to the dialysate drain line L2 through bypass lines L4 and L5. The bypass lines L4 and L5 are provided with electromagnetic valves V4 and V5, respectively.

The dialysate drain line L2 is provided with detour lines L3 and L6 that detour the duplex pump 7. The detour line L6 is provided with an electromagnetic valve V6. The detour line L3 is provided with an ultrafiltration pump 8 (an ultrafiltration device). Hence, when the ultrafiltration pump 8 is activated in the process of causing the blood of the patient to extracorporeally circulate through the blood circuit 2, ultrafiltration can be performed in which water is removed from the blood flowing through the dialyzer 3.

The dialysate drain line L2 is also provided with a pressurizing pump 9 on the upstream side (the right side in Fig. 1) with respect to the duplex pump 7. The pressurizing pump 9 adjusts the hydraulic pressure in the dialysate drain line 1.2 at the duplex pump 7. The dialysate drain line L2 is also provided with a detour line L7 connected thereto through a degassing chamber 10 at a position between the pressurizing pump 9 and the duplex pump 7. The dialysate drain line L2 and the detour line L7 extending therefrom are provided with respective electromagnetic valves V2 and V7, so that the dialysate flow route can be closed and opened with any timing.

A infusion line L8 (an infusion device) is connected at one end thereof to a collecting port (a sampling port) provided at a predetermined position of the dialysate introduction line L1 (in the present embodiment, between the electromagnetic valve V1 and the filter F2), and at the other end thereof to the arterial blood circuit 2a (on the upstream side with respect to the position of the blood pump 4 (the distal side of the arterial blood circuit 2a)). Thus, the infusion line L8 forms a flow route through which the dialysate in the dialysate introduction line L1 can be supplied to the arterial blood circuit 2a. The infusion line L8 is provided with an electromagnetic valve V8. When the electromagnetic valve V8 is open, the dialysate in the dialysate introduction line L1 can be supplied to the blood circuit 2 for fluid infusion or blood return.

The ultrafiltration pump 8 according to the present embodiment corresponds to the "ultrafiltration device capable of performing ultrafiltration for removing water from liquid in the blood circuit" according to the present invention. The infusion line L8 corresponds to the "infusion device capable of performing fluid infusion or blood return by introducing liquid into the blood circuit". That is, when the ultrafiltration pump 8 is activated, liquid, such as blood, in the blood circuit 2 is filtered in the dialyzer 3, whereby water can be removed from the liquid. Furthermore, the dialysate (infusion fluid) is introduced into the blood circuit 2 through the infusion line L8 for emergency fluid infusion, and blood return is performed by using the dialysate as a substitution fluid.

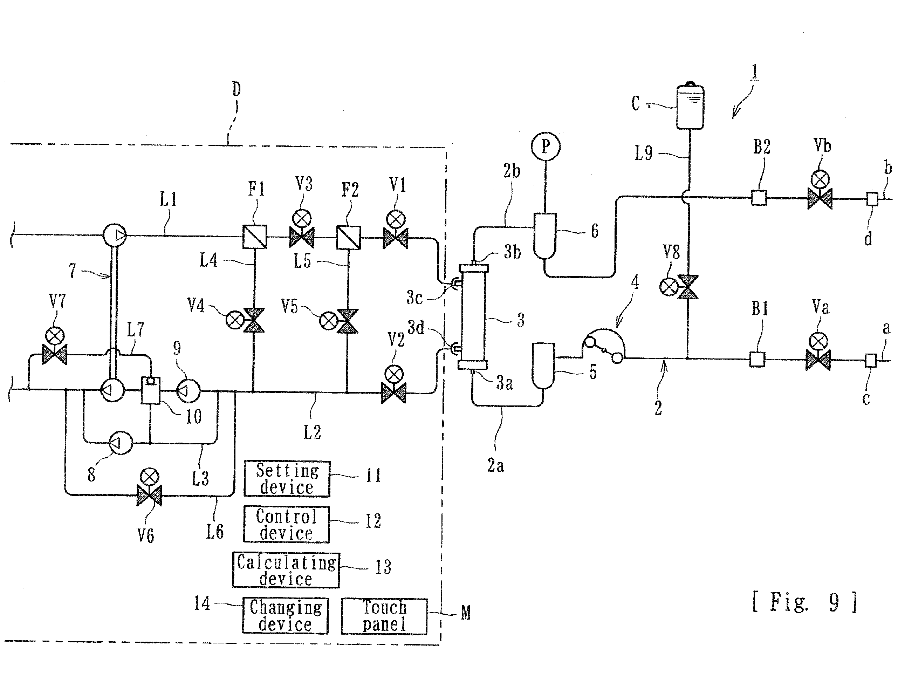

The ultrafiltration pump 8 may be replaced with an ultrafiltration device of another form that is capable of performing ultrafiltration by removing water from the liquid in the blood circuit 2. The infusion line L8 may be replaced with an infusion device of another form that is capable of performing fluid infusion or blood return by introducing liquid into the blood circuit 2. In particular, the infusion device of another form may be, as illustrated in Fig. 9, a saline supply line L9 provided with a storage bag C storing a physiological saline solution and with an electromagnetic valve V8 capable of opening and closing the flow route (in this case, fluid infusion or blood return is performed by using the physiological saline solution), or may be a device that performs fluid infusion by back-filtering the dialysate in the dialyzer 3 (by filtering the dialysate through filtration membranes into the blood circuit 2), for example.

The setting device 11 is capable of setting parameters related to ultrafiltration performed by the ultrafiltration pump 8 (the ultrafiltration device). The setting device 11 according to the present embodiment is capable of setting, before the blood purification treatment, the amount of ultrafiltration and the rate of ultrafiltration. Typically, the amount of ultrafiltration and the rate of ultrafiltration are set in accordance with the dry weight (DW) of the patient that is estimated from the patient's body weight measured before the blood purification treatment. In the present embodiment, the parameters related to ultrafiltration (the amount of ultrafiltration and the rate of ultrafiltration) are set in accordance with a control signal transmitted from a central monitoring device (not illustrated) connected to the blood purification apparatus 1. Alternatively, the parameters may be inputted through the touch panel M provided on the body D.

The control device 12 controls the ultrafiltration pump 8 (the ultrafiltration device) in accordance with the parameters (the amount of ultrafiltration and the rate of ultrafiltration) that are set by the setting device 11. Specifically, the control device 12 controls the operation of the ultrafiltration pump 8 during a period from the start of the blood purification treatment until the end of the blood purification treatment such that the amount of ultrafiltration and the rate of ultrafiltration become respective values that have been set in advance by the setting device 11.

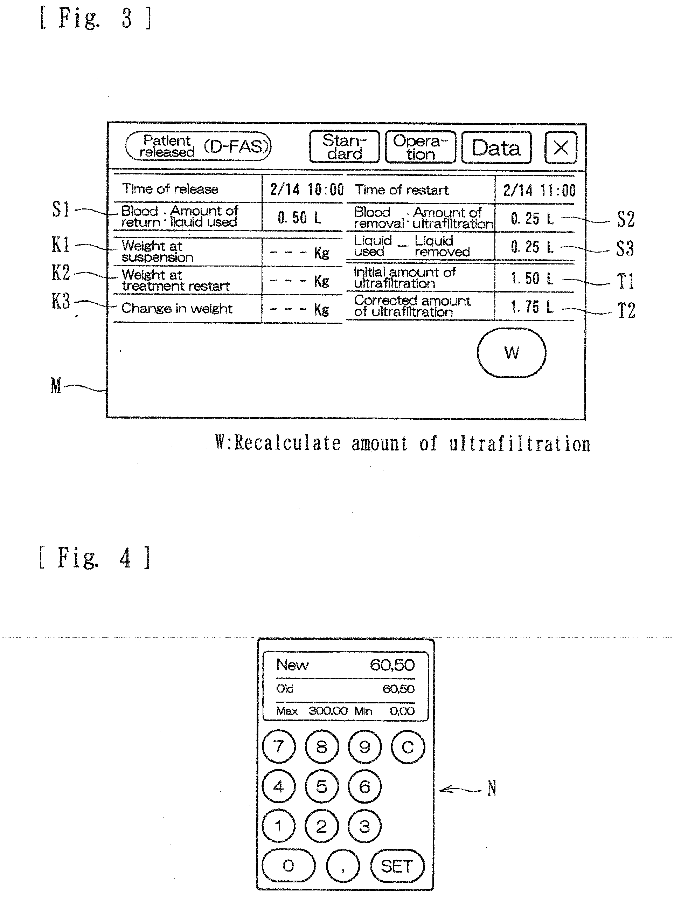

As illustrated in Fig. 2, the touch panel M is a display provided at the top of the body D and allows a person to touch any of predetermined parts of the screen thereof for making corresponding input. An input device N including a numeric keypad illustrated in Fig. 4 is provided on the body D (for example, near the touch panel M) according to the present embodiment. Hence, numerical values are inputtable through the input device N.

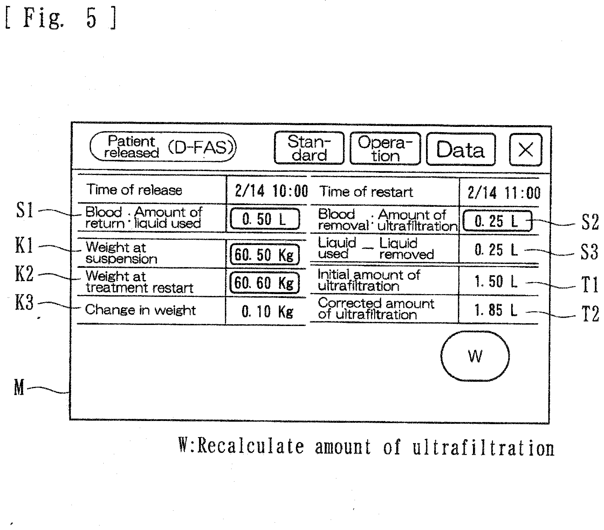

For example, as illustrated in Figs. 3 and 5, the screen of the touch panel M displays information on the blood purification treatment. A person touches any of the predetermined parts (items) and then inputs any numerical value through the input device N, whereby the value for the touched item (such as weight at suspension: K1, weight at treatment restart: K2, and change in weight: K3) can be selectively inputted. The input device N may be a separate input unit provided near the touch panel M, or an input portion displayed on the screen of the touch panel M. An input device of any form other than the numeric keypad is also acceptable.

The blood purification apparatus according to the present embodiment including the calculating device 13 and the changing device 14 is capable of restarting the blood purification treatment that has been suspended with the release of the patient from the blood circuit 2 (with the dislodgement of the arterial puncture needle a and the venous puncture needle b from the patient) during the blood purification treatment. Hence, a situation where, for example, the condition of the patient has changed significantly or the patient needs to take a bathroom break or the like during the blood purification treatment can be handled easily.

The expression "suspension and restart of the blood purification treatment" according to the present invention refers to the suspension of a particular treatment session and the restart of that treatment session. To make the treatment performed before the suspension and the treatment to be performed after the suspension the same as each other, the total amount of ultrafiltration performed before the suspension needs to be retained (stored in the blood purification apparatus) for the restart, and ultrafiltration in the treatment after the suspension needs to be restarted under the retained condition.

The calculating device 13 is capable of calculating variable amounts (a change in the patient's body weight, and various parameters affecting the change in the body weight) related to the patient's body weight that changes with the suspension and restart of the blood purification treatment. For example, the variable amounts related to the patient's body weight that are calculated by the calculating device 13 according to the present embodiment include the amount of liquid (infusion fluid or substitution fluid) delivered to the blood circuit 2 during fluid infusion or blood return performed through the infusion line L8 (the infusion device) at the suspension of the blood purification treatment, or the amount of water removed from the blood circuit 2 during ultrafiltration performed by the ultrafiltration pump 8 (the ultrafiltration device) in the process of blood removal at the restart of the blood purification treatment.

In addition, the blood purification apparatus 1

according to the present embodiment is configured such that, as illustrated in Figs. 3 and 5, a plurality of variable amounts related to the patient's body weight that changes with the suspension and restart of the blood purification treatment are displayable on one particular screen of the touch panel M (the display). The variable amounts related to the patient's body weight that are displayed on the touch panel M include, as illustrated in the drawings, at least the amount of liquid (infusion fluid or substitution fluid) delivered to the blood circuit 2 during fluid infusion or blood return performed through the infusion line L8 (the infusion device) at the suspension of the blood purification treatment (see item S1 in the drawings), and the amount of water removed from the blood circuit 2 during ultrafiltration performed by the ultrafiltration pump 8 (the ultrafiltration device) in the process of blood removal at the restart of the blood purification treatment (see item S2 in the drawings).

That is, to suspend the blood purification treatment, emergency fluid infusion is performed through the infusion line L8, according to need. Subsequently, substitution fluid is delivered into the blood circuit 2 for blood return. The amount of liquid thus delivered is displayed as item S1. Furthermore, to restart the blood purification treatment, the ultrafiltration pump 8 is activated for blood removal, whereby the substitution fluid or the like delivered as above for blood return can be drained from the dialyzer 3 into the dialysate drain line L2. In such a case, the amount of water removed by ultrafiltration is displayed as item S2.

Item S1 as "the amount of liquid used (the amount of liquid delivered) for blood return" and item S2 as "the amount of ultrafiltration for blood removal (the amount of water removed)" are each detectable by a sensor or the like included in the blood purification apparatus 1. If ultrafiltration is not performed at the time of blood removal, a numerical value "0" is displayed as item S2, and a numerical value that is the same as the value displayed as item S1 is displayed as item S3.

The particular screen (a patient-released screen) of the touch panel M displays the amount of liquid used (the amount of liquid delivered) as item S1, and the amount of ultrafiltration (the amount of water removed) as item S2. In addition, the calculating device 13 calculates the amount of water (liquid used - liquid removed) by subtracting the amount of ultrafiltration as item S2 from the amount of liquid used as item S1, and the calculated amount of water is displayed as item S3 on the screen. The particular screen (the patient-released screen) of the touch panel M also displays the time of release and the time of restart, and the amount of ultrafiltration that has been set before the blood purification treatment is started (the initial setting for the amount of ultrafiltration) as item T1.

The variable amounts related to the patient's body weight that are calculated by the calculating device 13 and the variable amounts related to the patient's body weight that are displayed on the touch panel M in the present embodiment include the amount of liquid (such as infusion fluid or substitution fluid) delivered through the infusion line L8, the increment in the body weight that is attributed to food and drink taken by the patient, the amount of water removed by the ultrafiltration pump 8, and the decrement in the body weight that is attributed to the urination or defecation by the patient.

Specifically, as illustrated in Fig. 3, the patient's body weight at the suspension of the blood purification treatment (item K1) and the patient's body weight at the restart of the blood purification treatment (item K2) are each inputtable by manual operation of touching of the item on the touch panel M and inputting the patient's measured body weight through the input device N. Furthermore, the change in the patient's body weight (item K3) is calculable from the inputted body weights and is displayable as illustrated in Fig. 5.

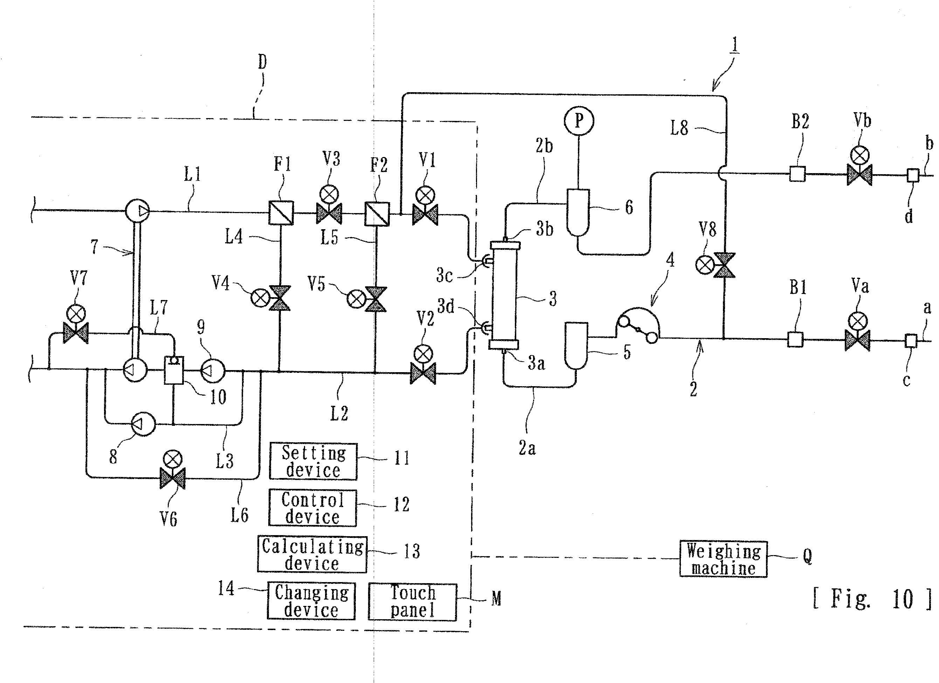

According to the present embodiment, any of the variable amounts related to the patient's body weight that are calculated by the calculating device 13 or displayed on the touch panel M are manually inputtable by an operator through the touch panel M or the input device N. Alternatively, such values may be inputted through a piece of equipment linked to the body D (such as a weighing machine Q connected to the body D as illustrated in Fig. 10). In that case, as illustrated in Fig. 5, the patient's body weights measured by the weighing machine Q before and after the treatment are automatically inputted as items K1 and K2, respectively, and the difference between the body weights before and after the treatment is calculated by the calculating device 13 and is displayed as item K3.

The changing device 14 is capable of changing, in accordance with the variable amounts calculated by the calculating device 13, the parameters related to ultrafiltration (in the present embodiment, the amount of ultrafiltration and the rate of ultrafiltration) that have been set by the setting device 11. For example, as illustrated in Figs. 3 and 5, a switch part W indicating "recalculate the amount of ultrafiltration" is provided on the particular screen (the patient-released screen) of the touch panel M. If the switch part W is touched for input, the calculating device 13 calculates the variable amounts related to the patient's body weight that changes with the suspension and restart of the blood purification treatment. In accordance with the results of the calculation, the changing device 14 can change the parameters related to ultrafiltration that have been set by the setting device 11.

If the parameters related to ultrafiltration that have been set by the setting device 11 are changed by the changing device 14, one (the corrected amount of ultrafiltration) of the changed parameters is displayed as item T2. Then, in accordance with the changed parameters (the amount of ultrafiltration and the rate of ultrafiltration), the control device 12 controls the ultrafiltration pump 8. Thus, if the switch part W is touched, the changing device 14 automatically changes the parameters related to ultrafiltration for the restart of the blood purification treatment. Hence, the occurrence of wrong setting due to wrong input can be prevented.

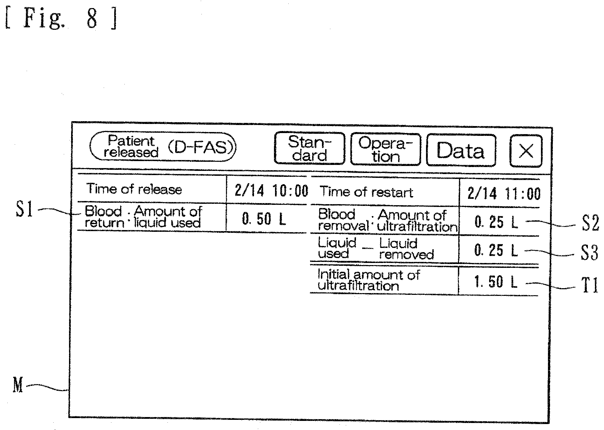

The touch panel M only needs to be capable of displaying, on one particular screen, a plurality of variable amounts related to the patient's body weight that changes with the suspension and restart of the blood purification treatment. Specifically, as illustrated in Fig. 8, at least the amount of liquid delivered to the blood circuit 2 during fluid infusion or blood return performed through the infusion line L8 (the infusion device) at the suspension of the blood purification treatment (item S1), and the amount of water removed from the blood circuit 2 during ultrafiltration performed by the ultrafiltration pump 8 (the ultrafiltration device) in the process of blood removal at the restart of the blood purification treatment (item S2) only need to be displayed simultaneously. In such a case, the switch part W is not displayed but may be displayed on the particular screen.

According to the present embodiment, the blood purification apparatus includes the calculating device 13 capable of calculating variable amounts related to the patient's body weight that changes with the suspension and restart of the blood purification treatment; and the changing device 14 capable of changing, in accordance with the variable amounts calculated by the calculating device 13, parameters related to ultrafiltration that are set by the setting device 11. Therefore, the parameters related to ultrafiltration (the amount of ultrafiltration and the rate of ultrafiltration) can be changed assuredly and smoothly at the restart of the blood purification treatment with the dialyzer 3 (the blood purifier) suspended with the release of the patient from the blood circuit 2 during the blood purification treatment.

Furthermore, the variable amounts related to the patient's body weight that are calculated by the calculating device 13 or displayed on the touch panel M (the display) include the amount of liquid delivered to the blood circuit 2 during fluid infusion or blood return performed through the infusion line L8 (the infusion device) at the suspension of the blood purification treatment, or the amount of water removed from the blood circuit 2 during ultrafiltration performed by the ultrafiltration pump 8 (the ultrafiltration device) in the process of blood removal at the restart of the blood purification treatment. Therefore, the parameters related to ultrafiltration can be changed more assuredly and smoothly.

Furthermore, the variable amounts related to the patient's body weight that are calculated by the calculating device 13 or displayed on the touch panel M (the display) include the amount of liquid delivered by the infusion device, the increment in the body weight that is attributed to food and drink taken by the patient, the amount of water removed by the ultrafiltration device, and the decrement in the body weight that is attributed to the urination or defecation by the patient. Therefore, the parameters related to ultrafiltration can be changed much more assuredly and smoothly.

Furthermore, the patient's body weight at the suspension of the blood purification treatment and the patient's body weight at the restart of the blood purification treatment are inputtable, and the change in the patient's body weight is calculable from the inputted body weights. Therefore, the parameters related to ultrafiltration can be changed in accordance with the change in the body weight that cannot directly be grasped by the blood purification apparatus. In particular, any of the variable amounts related to the patient's body weight that are calculated by the calculating device 13 or displayed on the touch panel M (the display) are inputtable through a piece of equipment (such as the weighing machine Q illustrated in Fig. 10) linked to the body D or through manual operation (performed on the input device N or the like illustrated in Fig. 4) by the operator. Therefore, the parameters related to ultrafiltration can be changed in accordance with any of the variable amounts related to the body weight that cannot directly be grasped by the blood purification apparatus 1.

In addition, the display according to the present embodiment is the touch panel M that allows a person to touch any of predetermined parts of the screen thereof for making corresponding input. When a person touches a display part representing any of the items on the screen displaying a plurality of variable amounts related to the patient's body weight, one of the variable amounts related to the patient's body weight that corresponds to the touched item can be inputted. Therefore, the work of inputting the variable amounts related to the body weight in changing the parameters related to ultrafiltration can be performed easily.

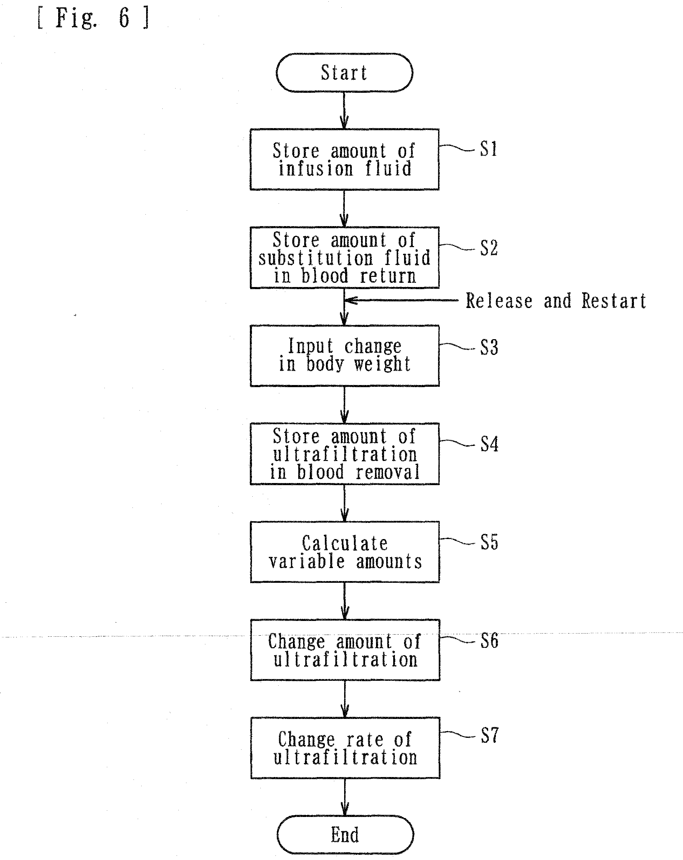

Now, a method of changing the parameters related to ultrafiltration by using the calculating device 13 and the changing device 14 according to the present embodiment will be described with reference to the flow chart illustrated in Fig. 6.

If emergency fluid infusion is necessary for suspending the blood purification treatment, the amount of infusion fluid introduced into the blood circuit 2 during the infusion is stored (S1). Subsequently, the amount of substitution fluid introduced into the blood circuit 2 during blood return is stored (S2). If neither emergency fluid infusion nor blood return is performed at the suspension, the amount of infusion fluid or the amount of substitution fluid is stored as "0".

Then, the patient is released from the blood circuit 2, and the blood purification treatment is suspended. Subsequently, when the blood purification treatment with the dialyzer 3 in which blood of the patient is caused to extracorporeally circulate through the blood circuit 2 is ready to be restarted, the patient-released screen is made to be displayed on the touch panel M (see Fig. 3). When this screen is displayed, items K1 and K2 are each touched with a finger. Then, numerical values (the body weight at the suspension and the body weight at the treatment restart) are each inputted through the input device N. Thus, the change in the body weight that is obtained as the difference between the two values is inputted as item K3 (S3).

Subsequently, the blood pump 4 is activated for blood removal. According to need, the ultrafiltration pump 8 is also activated for removing a predetermined amount of water from the blood circuit 2 by the effect of ultrafiltration, and the amount of ultrafiltration is stored (S4). If ultrafiltration is not performed during blood removal, the amount of ultrafiltration is stored as "0". Subsequently, the switch part W on the screen of the touch panel M is touched, whereby the calculating device 13 calculates the variable amounts related to the patient's body weight that changes with the suspension and restart of the blood purification treatment (S5). Consequently, as illustrated in Fig. 5, a plurality of variable amounts (items S1 to S3 and K1 to K3) are displayed on one particular screen.

The touching of the switch part W also causes the changing device 14 to change, in accordance with the variable amounts calculated by the calculating device 13, the parameters related to ultrafiltration that have been set by the setting device 11 (the amount of ultrafiltration is changed in step S6, and the rate of ultrafiltration is changed in step S7). In this case, the changed amount of ultrafiltration is displayed as item T2 (see Fig. 5). In the blood purification treatment thereafter, the control device 12 controls the ultrafiltration pump 8 in accordance with the changed parameters related to ultrafiltration (the amount of ultrafiltration and the rate of ultrafiltration).

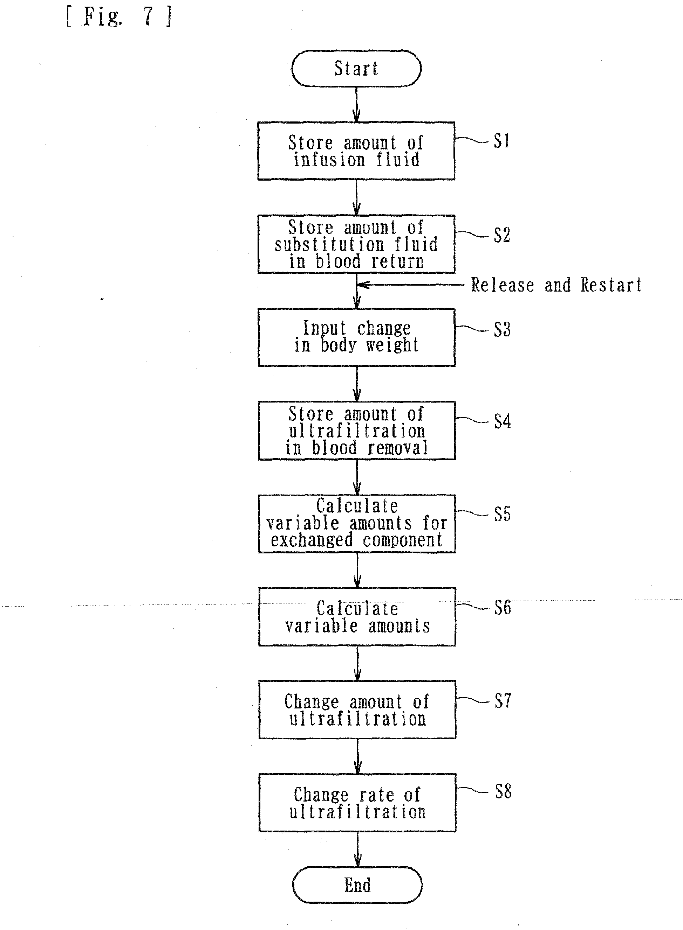

Now, a method of changing the parameters related to ultrafiltration by using the calculating device 13 and the changing-device 14 according to another embodiment will be described with reference to the flow chart illustrated in Fig. 7.

If emergency fluid infusion is necessary for suspending the blood purification treatment, the amount of infusion fluid introduced into the blood circuit 2 during the infusion is stored (S1). Subsequently, the amount of substitution fluid introduced into the blood circuit 2 during blood return is stored (S2). If neither emergency fluid infusion nor blood return is performed at the suspension, the amount of infusion fluid or the amount of substitution fluid is stored as "0".

Then, the patient is released from the blood circuit 2, and the blood purification treatment is suspended. Subsequently, when the blood purification treatment with the dialyzer 3 in which blood of the patient is caused to extracorporeally circulate through the blood circuit 2 is ready to be restarted, the patient-released screen is made to be displayed on the touch panel M (see Fig. 3). When this screen is displayed, items K1 and K2 are each touched with a finger. Then, numerical values (the body weight at the suspension and the body weight at the treatment restart) are each inputted through the input device N. Thus, the change in the body weight that is obtained as the difference between the two values is inputted as item K3 (S3).

Subsequently, the blood pump 4 is activated for blood removal. According to need, the ultrafiltration pump 8 is also activated for removing a predetermined amount of water from the blood circuit 2 by the effect of ultrafiltration, and the amount of ultrafiltration is stored (S4). If ultrafiltration is not performed during blood removal, the amount of ultrafiltration is stored as "0". In step S5 according to the present embodiment, the calculating device 13 calculates the variable amounts for exchanged parts.

For example, the total volume (priming volume) of the liquid flow route in the dialyzer 3 (the blood purifier) or the blood circuit 2 is inputtable through the input device N or the like. If the dialyzer 3 or the blood circuit 2 is exchanged at the restart of the blood purification treatment, the variable amounts related to the patient's body weight can be calculated in accordance with the inputted total volume. The dialyzer 3 or the blood circuit 2, which are disposable components, may be exchanged for various reasons after the suspension of the blood purification treatment. Even if such a disposable component is exchanged after the suspension and before the restart of the blood purification treatment, the variable amounts related to the patient's body weight can be calculated.

Subsequently, the switch part W on the screen of the touch panel M is touched, whereby the calculating device 13 calculates the variable amounts related to the patient's body weight that changes with the suspension and restart of the blood purification treatment (S6). Consequently, as illustrated in Fig. 5, a plurality of variable amounts (items S1 to S3 and K1 to K3) are displayed on one particular screen. The touching of the switch part W also causes the changing device 14 to change, in accordance with the variable amounts calculated by the calculating device 13, the parameters related to ultrafiltration that have been set by the setting device 11 (the amount of ultrafiltration is changed in step S7, and the rate of ultrafiltration is changed in step S8). In this case, the changed amount of ultrafiltration is displayed as item T2 (see Fig. 5). In the blood purification treatment thereafter, the control device 12 controls the ultrafiltration pump 8 in accordance with the changed parameters related to ultrafiltration (the amount of ultrafiltration and the rate of ultrafiltration).

According to the present embodiment, the total volume of the liquid flow route in the dialyzer 3 (the blood purifier) or the blood circuit 2 is inputtable. If the dialyzer 3 or the blood circuit 2 is exchanged at the restart of the blood purification treatment, the variable amounts related to the patient's body weight can be calculated in accordance with the inputted total volume. Therefore, even if the dialyzer 3 or the blood circuit 2 is exchanged at the restart of the blood purification treatment, the parameters related to ultrafiltration (the amount of ultrafiltration and the rate of ultrafiltration) can be changed assuredly.

While some embodiments have been described above, the present invention is not limited thereto. For example, the variable amounts related to the patient's body weight that are calculated by the calculating device 13 may be other variable amounts. Furthermore, while the display according to each of the above embodiments is the touch panel M provided on the body D, the display may alternatively be a monitor or the like that only displays information, or may be a device installed separately from the body D. Moreover, the parameters related to ultrafiltration that are to be changed by the changing device 14 may be parameters other than the amount of ultrafiltration and the rate of ultrafiltration.

Furthermore, according to each of the above embodiments, a plurality of variable amounts related to the patient's body weight that changes with the suspension and restart of the blood purification treatment are displayable on one particular screen of the touch panel M. Such a displaying method corresponds to the invention as claimed. In addition, the above embodiments each concern a hemodialysis apparatus capable of performing a treatment such as hemodialysis (HD), ECUM, or HDF (hemodiafiltration). Alternatively, the present invention may be applied to a blood purification apparatus capable of performing another kind of blood purification treatment (such as hemofiltration (HF), continuous slow hemofiltration (CHF), or the like).

### Industrial Applicability

The present invention is applicable to any blood purification apparatus, including those having different external shapes, other additional functions, and so forth, as long as the blood purification apparatus includes a calculating device capable of calculating a variable amount related to the patient's body weight that changes with the suspension and restart of the blood purification treatment; and a changing device capable of changing, in accordance with the variable amount calculated by the calculating device, a parameter related to ultrafiltration that is set by a setting device.

### Reference Signs List

- 1: blood purification apparatus
- 2: blood circuit
- 3: dialyzer (blood purifier)
- 4: blood pump
- 5: arterial air-trap chamber
- 6: venous air-trap chamber
- 7: duplex pump
- 8: ultrafiltration pump (ultrafiltration device)
- 9: pressurizing pump
- 10: degassing chamber
- 11: setting device
- 12: control device
- 13: calculating device
- 14: changing device
- L1: dialysate introduction line
- L2: dialysate drain line
- L8: infusion line (infusion device)
- M: touch panel (display)
- D: body
- N: input device

## Claims

1. A blood purification apparatus (1) comprising:
a blood circuit (2) through which blood of a patient is allowed to extracorporeally circulate;
a blood purifier (3) for giving blood purification treatment by purifying the blood of the patient that extracorporeally circulates through the blood circuit (2);
an ultrafiltration device (8) capable of performing ultrafiltration for removing water from liquid in the blood circuit (2);
an infusion device (L8) capable of performing fluid infusion or blood return by introducing liquid into the blood circuit (2);
a setting device (11) capable of setting a parameter related to ultrafiltration performed by the ultrafiltration device (8); and
a control device (12) that controls the ultrafiltration device (8) in accordance with the parameter that is set by the setting device (11),
wherein the blood purification treatment that is suspended with a release of the patient from the blood circuit (2) during the blood purification treatment is restartable,
wherein the blood purification apparatus (1) further includes
a calculating device (13) capable of calculating a variable amount related to the patient's body weight that changes with the suspension and restart of the blood purification treatment;
a changing device (14) capable of changing, in accordance with the variable amount calculated by the calculating device (13), the parameter related to ultrafiltration that is set by the setting device (11); and
a display (M), wherein the blood purification apparatus (1) is configured such that a plurality of variable amounts related to the patient's body weight that changes with the suspension and restart of the blood purification treatment are displayable on one particular screen of the display (M).

2. The blood purification apparatus (1) according to Claim 1, wherein the variable amount related to the patient's body weight that is calculated by the calculating device (13) is one of variable amounts including an amount of liquid delivered to the blood circuit (2) during fluid infusion or blood return performed by the infusion device (L8) at the suspension of the blood purification treatment, or an amount of water removed from the blood circuit (2) during ultrafiltration performed by the ultrafiltration device (8) in a process of blood removal at the restart of the blood purification treatment.

3. The blood purification apparatus (1) according to Claim 2, wherein the variable amount related to the patient's body weight that is calculated by the calculating device (13) is one of variable amounts including the amount of liquid delivered by the infusion device (L8), an increment in the body weight that is attributed to food and drink taken by the patient, the amount of water removed by the ultrafiltration device (8), and a decrement in the body weight that is attributed to urination or defecation by the patient.

4. The blood purification apparatus (1) according to Claim 3, wherein the patient's body weight at the suspension of the blood purification treatment and the patient's body weight at the restart of the blood purification treatment are inputtable, and a change in the patient's body weight is calculable from the inputted body weights.

5. The blood purification apparatus (1) according to any of Claims 1 to 4, wherein any variable amount related to the patient's body weight that is calculated by the calculating device (13) is inputtable through a piece of equipment linked to a body (D) of the apparatus or through manual operation by an operator.

6. The blood purification apparatus (1) according to any of Claims 1 to 5, wherein a total volume of a liquid flow route in the blood purifier (3) or the blood circuit (2) is inputtable, and wherein if the blood purifier (3) or the blood circuit (2) is exchanged at the restart of the blood purification treatment, the variable amount related to the patient's body weight is calculable in accordance with the inputted total volume.

## Patentansprüche

1. Ein Blutreinigungsapparat (1), umfassend:
einen Blutkreislauf (2), durch den das Blut eines Patienten extrakorporal zirkulieren kann;
einen Blutreiniger (3) zur Durchführung einer Blutreinigungsbehandlung durch Reinigung des extrakorporal durch den Blutkreislauf (2) zirkulierenden Blutes des Patienten;
ein Ultrafiltrationsgerät (8), das in der Lage ist, eine Ultrafiltration durchzuführen, um Wasser aus der Flüssigkeit im Blutkreislauf (2) zu entfernen;
ein Infusionsgerät (L8), das in der Lage ist, eine Fluidinfusion oder einen Blutrückfluss durch Einleiten von Flüssigkeit in den Blutkreislauf (2) durchzuführen;
eine Einstellvorrichtung (11), die in der Lage ist, einen Parameter einzustellen, der die von dem Ultrafiltrationsgerät (8) durchgeführte Ultrafiltration betrifft; und
eine Steuervorrichtung (12), die das Ultrafiltrationsgerät (8) in Übereinstimmung mit dem Parameter steuert, der durch die Einstellvorrichtung (11) eingestellt wird,
wobei die Blutreinigungsbehandlung, die mit einer Entlassung des Patienten aus dem Blutkreislauf (2) während der Blutreinigungsbehandlung unterbrochen wird, wieder neu gestartet werden kann,
wobei der Blutreinigungsapparat (1) ferner einschließt
eine Rechenvorrichtung (13), die in der Lage ist, einen variablen Betrag zu berechnen, der sich auf das Körpergewicht des Patienten bezieht und sich mit der Unterbrechung und dem Neustart der Blutreinigungsbehandlung ändert;
eine Änderungsvorrichtung (14), die in der Lage ist, in Übereinstimmung mit dem von der Rechenvorrichtung (13) berechneten variablen Betrag den auf die Ultrafiltration bezogenen Parameter zu ändern, der von der Einstellvorrichtung (11) eingestellt wird; und
eine Anzeige (M), wobei der Blutreinigungsapparat (1) so ausgebildet ist, dass eine Vielzahl von variablen Beträgen, die sich auf das Körpergewicht des Patienten beziehen, das sich mit dem Aussetzen und Neustart der Blutreinigungsbehandlung ändert, auf einem bestimmten Bildschirm der Anzeige (M) anzeigbar sind.

2. Blutreinigungsapparat (1) nach Anspruch 1, wobei der variable Betrag, der sich auf das Körpergewicht des Patienten bezieht und von der Rechenvorrichtung (13) berechnet wird, einer der variablen Beträge ist, die eine Flüssigkeitsmenge einschließen, die dem Blutkreislauf (2) während einer Fluidinfusion oder einer Blutrückführung zugeführt wird, die von dem Infusionsgerät (L8) bei der Aussetzung der Blutreinigungsbehandlung durchgeführt wird, oder eine Wassermenge, die aus dem Blutkreislauf (2) während der Ultrafiltration entfernt wird, die von dem Ultrafiltrationsgerät (8) in einem Verfahren zur Blutentfernung beim Neustart der Blutreinigungsbehandlung durchgeführt wird.

3. Blutreinigungsapparat (1) nach Anspruch 2, wobei der variable Betrag, der sich auf das Körpergewicht des Patienten bezieht und von der Rechenvorrichtung (13) berechnet wird, einer der variablen Beträge ist, die die von dem Infusionsgerät (L8) abgegebene Flüssigkeitsmenge, eine Zunahme des Körpergewichts, die auf vom Patienten eingenommene Nahrung oder Getränke zurückzuführen ist, die von dem Ultrafiltrationsgerät (8) entnommene Wassermenge und eine Abnahme des Körpergewichts, die auf das Urinieren oder die Defäkation des Patienten zurückzuführen ist, umfassen.

4. Blutreinigungsapparat (1) nach Anspruch 3, wobei das Körpergewicht des Patienten bei Aussetzung der Blutreinigungsbehandlung und das Körpergewicht des Patienten beim Neustart der Blutreinigungsbehandlung eingebbar sind und eine Änderung des Körpergewichts des Patienten aus den eingegebenen Körpergewichten berechenbar ist.

5. Blutreinigungsapparat (1) nach einem der Ansprüche 1 bis 4, wobei jeder variable Betrag, der sich auf das Körpergewicht des Patienten bezieht und von der Rechenvorrichtung (13) berechnet wird, über ein mit einem Körper (D) des Apparates verbundenes Gerät oder durch manuelle Bedienung durch einen Bediener eingegeben werden kann.

6. Blutreinigungsapparat (1) nach einem der Ansprüche 1 bis 5, wobei ein Gesamtvolumen einer Strömungsleitung im Blutreiniger (3) oder im Blutkreislauf (2) eingebbar ist, und wobei, wenn der Blutreiniger (3) oder der Blutkreislauf (2) beim Neustart der Blutreinigungsbehandlung ausgetauscht wird, der auf das Körpergewicht des Patienten bezogene variable Betrag in Übereinstimmung mit dem eingegebenen Gesamtvolumen berechenbar ist.

## Revendications

1. Appareil (1) de purification de sang comprenant :
un circuit sanguin (2) qui permet de circuler le sang d'un patient de manière extracorporelle ;
des moyens (3) de purification de sang pour donner un traitement de purification de sang en purifiant le sang du patient qui circule de manière extracorporelle dans le circuit sanguin (2) ;
un dispositif d'ultrafiltration (8) apte à effectuer une ultrafiltration pour extraire de l'eau du liquide dans le circuit sanguin (2) ;
un dispositif d'infusion (L8) apte à effectuer une infusion de fluide ou un retour de sang en introduisant du liquide dans le circuit sanguin (2) ;
un dispositif de configuration (11) apte à configurer un paramètre concernant une ultrafiltration exécutée par ledit dispositif d'ultrafiltration (8) ; et
un dispositif de commande (12) destiné à commander ledit dispositif d'ultrafiltration (8) en fonction du paramètre configuré par ledit dispositif de configuration (11),
un traitement de purification de sang qui est interrompu par un détachement du patient du circuit sanguin (2) pendant le traitement de purification de sang étant apte à être repris, ledit appareil (1) de purification de sang comprenant en outre
un dispositif de calcul (13) apte à calculer une quantité variable concernant le poids corporel du patient qui varie avec l'interruption et la reprise du traitement de purification de sang ;
un dispositif de modification (14) apte à modifier, en fonction de la quantité variable calculée par le dispositif de calcul (13), le paramètre concernant l'ultrafiltration qui est configuré par ledit dispositif de configuration (11) ; et
un dispositif d'affichage (M), ledit appareil (1) de purification de sang étant configuré de façon qu'une pluralité de quantités variables concernant le poids corporel du patient qui varie avec l'interruption et la reprise du traitement de purification de sang peuvent être affichées sur un écran particulier du dispositif d'affichage (M).

2. Appareil (1) de purification de sang selon la revendication 1, dans lequel la quantité variable concernant le poids corporel du patient qui est calculée par ledit dispositif de calcul (13) est une de quantités variables comportant une quantité de liquide fournie au circuit sanguin (2) pendant une infusion de fluide ou un retour de sang effectué par le dispositif d'infusion (L8) lors de l'interruption du traitement de purification de sang, ou une quantité d'eau extraite du circuit sanguin (2) pendant l'ultrafiltration effectuée par ledit dispositif d'ultrafiltration (8) dans un processus d'extraction de sang lors de la reprise du traitement de purification de sang.

3. Appareil (1) de purification de sang selon la revendication 2, dans lequel la quantité variable concernant le poids corporel du patient qui est calculée par ledit dispositif de calcul (13) est une de quantités variables comportant la quantité de liquide fournie par le dispositif d'infusion (L8), une augmentation du poids corporel attribuée à la nourriture et les boissons prises par le patient, la quantité d'eau extraite par le dispositif d'ultrafiltration (8), et une réduction du poids corporel attribuée à une miction ou une défécation du patient.

4. Appareil (1) de purification de sang selon la revendication 3, dans lequel le poids corporel du patient lors de l'interruption du traitement de purification de sang et le poids corporel du patient lors de la reprise du traitement de purification de sang peuvent être entrés, et une variation du poids corporel du patient peut être calculée à partir des poids corporels entrés.

5. Appareil (1) de purification de sang selon l'une quelconque des revendications 1 à 4, dans lequel toute quantité variable concernant le poids corporel du patient qui est calculée par ledit dispositif de calcul (13) peut être entrée moyennant une pièce d'équipement connectée à un corps (D) de l'appareil ou moyennant une opération manuelle de l'opérateur.

6. Appareil (1) de purification de sang selon l'une quelconque des revendications 1 à 5, dans lequel le volume total de la voie d'écoulement du liquide dans les moyens (3) de purification de sang ou dans le circuit sanguin (2) peut être entré, et dans lequel, si les moyens (3) de purification de sang ou le circuit sanguin (2) sont remplacés lors de la reprise du traitement de purification de sang, la quantité variable concernant le poids corporel du patient peut être calculée en fonction du volume total entré.
